# EUROPÄISCHE PATENTANMELDUNG

(11) **EP 1 078 634 A1**
(43) Veröffentlichungstag der Anmeldung: **28.02.2001**
(21) Anmeldenummer: 99810750.2
(22) Anmeldetag: 20.08.1999
(51) Int. Cl.: A61K 31/519, A61P 29/00, A61P 25/28, A61P 35/00, A61P 43/00

(54) **Arzneimittel zur Hemmung von NF-kB**

(71) Anmelder: Max Zeller Söhne AG, 8590 Romanshorn (CH)
(72) Erfinder: Hamburger, Matthias, Prof. Dr., 07743 Jena (DE); Danz, Henning, 07743 Jena (DE)
(74) Vertreter: Ritscher, Thomas, Dr.

(57) **Zusammenfassung**

Indolo[2,1-b]chinazolin-6,12-dione der Formel (1) 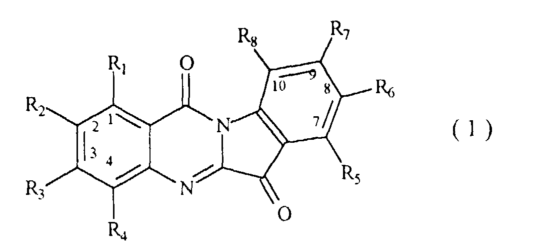 in der R₁ bis R₈ unabhängig voneinander gewählt sind aus Wasserstoffatomen und pharmazeutisch akzeptablen Substituenten, und pharmazeutisch akzeptable Salze von Verbindungen der Formel (1) zur Verwendung für Arzneimittel zur Hemmung des Trans-kriptionsfaktors NF-kB und/oder zur Hemmung von COX mit einer präferentiellen Hemmwirkung auf COX-2; solche Arzneimittel eigenen sich zur Verwendung als antiphlogistische, insbesondere antirheumatische Mittel zur Behandlung von entzündlichen Erkrankungen, insbesondere des rheumatischen Formenkreises und der Arthritiden, sowie zur präventiven oder adjuvanten Behandlung von Krebs und morbus Alzheimer sowie für die präventive oder adjunktive Dauermedikation gegen Altersbeschwerden.

## Beschreibung

Die vorliegende Erfindung betrifft neue Arzneimittel zur Hemmung des Transkriptionsfaktors NF-kB (kurz auch einfach NF-kB genannt) und der Cyclooxygenasen unter präferentieller Hemmung der Cyclooxygenase-2.

Transkriptionsfaktoren sind Proteine, die durch eine spezifische Bindung an bestimmte DNA-Abschnitte das Ablesen der codierten Erbinformation induzieren können. Bei verschiedenen entzündlichen Prozessen spielt insbesondere der Transkriptionsfaktor NF-kB eine wichtige Rolle, da viele entzündungsfördernde Induktoren zu seiner Aktivierung führen. Der aktivierte Transkriptionsfaktor seinerseits führt zur Expression von verschiedenen Genprodukten, die ihrerseits die Synthese entzündungsfördernder Substanzen katalysieren. Von besonderer Bedeutung ist die Cyclooxygenase-2 (COX-2), auch als induzierbare COX bezeichnet, die das Schlüsselenzym in der Synthese von entzündungsfördernden Prostaglandinen ist (siehe Baeuerle, D. und Baltimore, D., Cell 87, 141-179 (1994); Baldwin, Jr. A.S., Annu. Rev. Immuniol. 14, 649-683 (1996)). Neben klassischen akut oder chronisch entzündlichen Prozessen ist NF-kB nach heutigem Stand des Wissens auch in weitere Krankheitsbilder wie z.B. morbus Alzheimer impliziert.

In den letzten Jahren sind einige Naturstoffe und Naturstoffabkömmlinge mit NF-kB hemmender Wirkung beschrieben worden. Dazu gehören die Curcuminoide aus der javanischen Gelbwurzel Curcuma longa, Kaffeesäurederivate aus Propolis (Bienenkittharz), Acetylsalicylsäure und Natriumsalicylat, das Alkaloid Tetrandrin sowie einige Sesquiterpenlactone (siehe Schmitz, M.L., Hehner, S.P., Bacher, S., Dröge, W., Heinrich, M., Deutsche Apoth. Ztg. 138, 4881-4891 (1998). Die Hemmwirkung der Curcuminoide und der Kaffeesäurederivate ist aber schwach und scheint durch die Radikalfängerdurch die Radikalfänger-Eigenschaften der Verbindungen zustande zu kommen. Die Hemmwirkung der Salicylate ist ebenfalls schwach. Die NF-kB inhibierende Wirkung der Sesquiterpenlactone ist mit der Anwesenheit einer exocyclischen α-ständigen Methylengruppe an einem γ-Lactonring verknüpft. Die Methylengruppe geht aufgrund ihrer Reaktivität leicht kovalente Bindungen mit nukleophilen Gruppen (z.B. SH-Gruppen von Cystein) ein.

Wirkstoffe mit inhibierender Wirkung auf NF-kB bieten jedoch einen neuartigen Ansatz zur Beeinflussung des Entzündungsgeschehens und können zur Hemmung akuter und chronischer entzündlicher Prozesse, als Antirheumatika sowie zur Prävention und Behandlung von neurodegenerativen Erkrankungen eingesetzt werden.

Die Cyclooxygenasen (auch kurz COX genannt) zählen zu den Schlüsselenzymen in der Biosynthese der Eicosanoide und katalysieren die Umsetzung von Arachidonsäure zu den kurzlebigen Prostaglandinen G und H, die durch spontanen Zerfall und weitere enzymatische Reaktionen in eine Vielzahl von physiologisch wirksamen Prostanoiden umgesetzt werden. Eicosanoide sind parakrine Hormone, die sich von C₂₀-Fettsäuren (Arachidonsäure) ableiten. Wichtige acyclische Eicosanoide sind die Leukotriene und Lipoxine; cyclische Derivate sind die Prostaglandine und Thromboxane. Unter dem Oberbegriff "Prostanoide" werden allgemein Verbindungen zusammengefasst, die sich von PGH₂ (Prostaglandin H₂) ableiten, das seinerseits durch COX aus Arachidonsäure gebildet wird. Die Eicosanoide haben vielerlei biologische Wirkungen, deren physiologische Basis wegen der Vielzahl von Strukturen und den teilweise gewebeabhängigen Wirkungen noch nicht vollständig geklärt sind (siehe z.B. Herschmann, H.R.; Biochim.Biophys. Acta 1299 (1996) 125 - 140).

Die Prostaglandine und Thromboxane spielen als Gewebshormone in einer Vielzahl von physiologischen und pathologischen Prozessen eine Rolle, unter anderem als Entzündungsmediatoren. Eine Hemmung der COX führt deshalb zur Reduktion pro-inflammatorischer Prostanoide. Dieser Mechanismus ist bei den nicht-steroidalen Antirheumatika (NSAIDs) für die anti-inflammatorischen und analgetischen Wirkungen, aber auch für zahlreiche Nebenwirkungen auf den Magen-Darm-Trakt sowie die Nieren und für die Blutgerinnung verantwortlich.

Arzneistoffe mit inhibierender Wirkung auf Cyclooxygenasen können klinisch als Entzündungshemmer eingesetzt werden. Dazu gehören die nicht-steroidalen Antirheumatika (NSAID's), die sich in ihrer Struktur erheblich unterscheiden können und mehr auf Grund ihrer ähnlichen pharmakologischen Wirkung eine Kategorie bilden. Typische bekannte Vertreter sind Acetylsalicylsäure (Ass), (3-Chlor-4-3-pyrrolin-1-yl)-phenylpropionsäure (Pirprofen),(S)-2-(6-Methoxy-2-naphthyl-3-pyrrolessigsäure (Naproxen),[2-(2,6-Dichloranilino)-phenyl]-essigsäure (Diclofenac) und [1-(4-Chlorbenzoyl)-5-methoxy-2-methyl-3-indolyl]-essigsäure (Indometacin), die jeweils in Form der freien Säuren oder als deren Salze verwendet werden.

Im Zusammenhang mit dem Interesse an Cyclooxygenase wurden in den letzten Jahren zwei Isozyme der Cyclooxygenase identifiziert. Nach heutigem Verständnis wird die Cyclooxygenase 1 (auch COX-1 bezeichnet) konstitutiv in vielen Zellen exprimiert und sorgt für die Synthese von Prostaglandinen und anderen Mediatoren, die in verschiedenen physiologischen Prozessen eine Rolle spielen, u.a. für die Aufrechterhaltung einer intakten Magenschleimhaut und einer normalen Nierenfunktion sowie der Aggregation der Blutplättchen. Cyclooxygenase 2 (oder kurz COX-2 genannt) hingegen kommt in den verschiedenen Geweben nicht oder nur in geringerem Masse konstitutiv vor, wird aber im Zuge von entzündlichen Prozessen verstärkt exprimiert. Cyclooxygenase 2 wird deshalb auch vereinfachend als induzierbare Cyclooxygenase bezeichnet.

Nach gegenwärtigem Stand des Wissens hat COX-2 physiologische Funktionen in der Niere, im Gehirn/Rückenmark und im weiblichen Reproduktionssystem, pathologische Funktionen in Entzündungsprozessen, bei Schmerz, morbus Alzheimer und Krebs (siehe z.B. Vane, J.R., et al., "Cyclooxygenases 1 and 2", Annu. Rev. Pharmacol. Toxicol. 38 (1998) 97-129).

Die Wirkung der NSAIDs als Antirheumatika bzw. antiinflammatorische Mittel kommt durch eine nicht-selektive Hemmung der COX zustande. Unerwünschte Nebenwirkungen, insbesondere Erosion der Magenschleimhaut und Beeinflussung der Blutgerinnung, sind im wesentlichen der von Cyclooxygenase 1 verursachten Hemmung der Synthese physiologisch wichtiger Prostaglandine zuzuschreiben.

Von Stoffen mit selektiver, und zwar präferentieller Hemmwirkung auf die induzierbare Cyclogenase, d.h. Cyclooxygenase 2, sind somit geringere Nebenwirkungen bei vergleichbarer Wirkung als Antiphlogistika bzw. Antirheumatika zu erwarten, weil mit diesen Stoffen die für eine Homöostase wichtigen Eicosanoide nicht oder in vermindertem Masse beeinflusst würden. Als "präferentielle Hemmung" wird im vorliegenden Zusammenhang eine relativ stärkere Hemmung verstanden, wobei sich diese Stärke in physiologisch signifikanter Weise äussert.

Im vorliegenden Zusammenhang werden die Bezeichnungen antiphlogistisch und antiinflammatorisch als synonyme allgemeinere Wirkungsbezeichnungen und antirheumatisch als speziellere Wirkungsbezeichnung verwendet, die von den allgemeinen generischen Bezeichnungen regelmässig umfasst wird. Mit anderen Worten: eine antirheumatisch wirkende Substanz ist immer auch antiphlogistisch oder antiinflammatorisch, aber nicht notwendigerweise auch umgekehrt wirksam.

Aufgabe der Erfindung ist es, ein neues Arzneimittel zur NF-kB Hemmung und/oder zur direkten oder indirekten Hemmung der Cyclooxygenasen mit Präferenz für die Cyclooxygenase 2 zu bieten.

Es wurde gefunden, dass sich diese Aufgabe durch das natürlich vorkommende, aber auch synthetisch zugängliche und z. B. aus WO 95/13807 als Mittel zur Hemmung von Mycobacterium tuberculosis beschriebene Tryptanthrin (Formel 1, in der R₁ bis R₈ Wasserstoffatome darstellen; systematische Bezeichnung Indolo[2,1-b]chinazolin-6,12-dion) und geeignet substituierte Derivate dieser Verbindung sowie geeignete Salze dieser Verbindungen lösen lässt.

Die Erfindung betrifft daher Indolo[2,1-b]chinazolin-6,12-dione, d.h. Verbindungen der Formel (1) 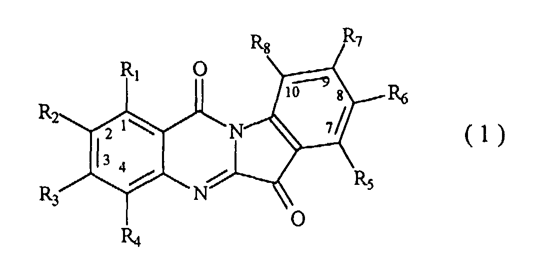 in der R₁ bis R₈ unabhängig voneinander gewählt sind aus Wasserstoffatomen und pharmazeutisch akzeptablen Substituenten, sowie die pharmazeutisch akzeptablen Salze der Verbindungen der Formel (1) zur Verwendung für die Herstellung von Arzneimitteln gemäss den Ansprüchen 1 - 5.

Gemäss einer weiteren Ausführungsform betrifft die Erfindung Arzneimittel mit Hemmwirkung auf den Transkriptionsfaktor NF-kB und/oder der Cyclooxygenasen unter präferentieller Hemmung der Cyclooxygenase 2 sowie zur Behandlung von chronischen oder akuten Krankheitszuständen, die sich durch eine solche Hemmwirkung positiv beeinflussen lassen und die dadurch gekennzeichnet sind, dass sie mindestens eine Verbindung der oben angegebenen Formel (1) oder ein pharmazeutisch akzeptables Salz einer solchen Verbindung enthalten.

Tryptanthrin (Couropitine A, Indolo[2,1-b]chinazolin-6,12-dion; Formel 1, R₁ bis R₈ = H) ist ein Alkaloid, welches schon seit mehreren Jahrzehnten bekannt ist und bis heute in einigen Pflanzen (Strobilanthus cusia, Polygonum tinctorium, Isatis tinctoria, Courcoupita guianensis, Wrightia tinctoria, Calanthe spec.) und Mikroorganismen (Candida lipolytica) nachgewiesen und isoliert werden konnte. Ob das natürlich vorkommende oder das synthetisch erhaltene Tryptanthrin erfindungsgemäss oder als Ausgangsstoff für die Herstellung von pharmazeutisch akzeptablen Derivaten bzw. Salzen verwendet wird, ist im wesentlichen eine Frage der Wirtschaftlichkeit.

Tryptanthrin und verschiedene Derivate wurden schon auf antimikrobielle Aktivität geprüft und wie oben erwähnt zur Inhibierung von Mycobacterium tuberculosis zusammen mit Synthesen zur Herstellung dieser Stoffe vorgeschlagen. Die Wirkung dieser Verbindungen auf die Hemmung des Transkriptionsfaktors NF-kB und/oder der Cyclooxygenasen unter präferentieller Hemmung der Cyclooxygenase 2 ist jedoch nach Wissen der Anmelderin bisher nicht bekannt geworden und war aufgrund des Standes der Technik auch nicht naheliegend.

Die erfindungsgemäss eingesetzten Verbindungen der obigen Formel (1) werden gemäss einer bevorzugten Ausführungsform in Form des auch natürlich vorkommenden Stoffes Tryptanthrin (Formel 1, alle R₁ bis R₈ sind Wasserstoffatome), gewünschtenfalls in Form eines pharmazeutisch akzeptablen Salzes, verwendet.

Eine oder mehrere von R₁ bis R₈ können pharmazeutisch akzeptable Substituenten darstellen, die vorzugsweise aus folgenden Gruppen gewählt sind: Halogen (F, Cl, Br), C₁ - C₁₀ Alkyl (Methyl bis Decyl, linear und verzweigt, gegebenenfalls durch Hydroxyl und/oder Halogen substituiert und/oder mit einer durch ein oder mehrere Sauerstoffatome unterbrochenen Kette), C₅ - C₆ Cycloalkyl, gegebenenfalls ringsubstituierte Heterozyklen mit 6 bis 10 C-Atomen, Amino, Imino, Nitro, C₁ - C₁₀ Alkylsulfonyl, C₆ - C₁₀ Aryl, Phenyl, Arylalkyl (wobei die Alkyl- und Arylgruppen der obigen Definition entsprechen), Arylalkylaryl (wobei die Alkyl- und Arylgruppen der obigen Definition entsprechen), C₆- C₁₀ Aryloxy, C₆ - C₁₀ Arylamino, Acylamino mit 1 - 10 C-Atomen in der Acylgruppe, Acyloxyamino mit 1 - 10 C-Atomen in der Acylgruppe, Alkylaminoacylamino mit insgesamt 1 - 10 C-Atomen, C₁ - C₁₀ Alkylaminosulfonylamino, C₁ - C₁₀ Alkylamino, C₁ - C₁₀ Alkenylamino, C₁ - C₁₀ Dialkylamino, C₁ - C₁₀ Alkoxyalkylamino, Cyano, Formyl, COOR₁₁ , wobei R₁₁ Wasserstoff, C₁ - C₁₀ Alkyl, C₆ - C₁₀ Aryl, Heterocyclen mit insgesamt maximal 12 C-Atomen oder Mono- oder Di-saccharid ist, und COONR₁₂R₁₃ , wobei R₁₂ und R₁₃ unabhängig voneinander die oben genannten Substituenten oder Aminosäure oder Peptid bedeuten können.

Pharmazeutisch akzeptable Salze von Verbindungen der Formel (1) sind Salze mit anorganischen oder organischen Säuren, wie typisch Salzsäure, Schwefelsäure, Phoshorsäure, Citronensäure, Milchsäure und dergleichen.

Die Verbindungen der Formel (1) bzw. deren pharmazeutisch akzeptable Salze können mit den zur Herstellung von flüssigen oder festen Arzenimittelzubereitungen üblichen Adjuvantien, Streckmittel, Trägern, Tablettierungshilfsmittel, Aromastoffen und dergleichen in üblicher Weise verarbeitet werden. Typische Dosierungen werden im Bereich von 1 - 100 mg /Tag liegen, doch ist die Obergrenze voraussichtlich nicht kritisch, da bisher in den relevanten Bereichen keine Toxizität für Warmblüter festgestellt werden konnte.

Die Erfindung wird nun anhand der folgenden Beispiele weiter erläutert.

### Beispiel 1

Die Isolierung erfolgt weitgehend nach der Vorschrift von Honda et al, M. Planta Medica 38 (1980) 275 - 276. Beispielhaft wird hier die Gewinnung aus Isatis tinctoria L beschrieben. Die Isolierung aus den oben genannten Organismen kann in analoger Weise durchgeführt werden.

Ein kg getrockneter gepulverter Droge wurde mit jeweils 3 x 10 Liter Methanol (+2% Essigsäure) 24 Stunden erschöpfend extrahiert, der Extrakt zur Trockne eingeengt (240 g) und in Wasser (mit 1% Ammoniak) aufgenommen. Anschliessend wurde mit Dichlormethan ausgeschüttelt und der erhaltene Extrakt erneut zur Trockne eingeengt (30 g).

Der Extrakt wurde über eine Silicagelsäule stufenweise mit Hexan-Chloroform 9:1, 1:2, Chloroform und Ethylacetat zerlegt, die Chloroformfraktion mit Säulenchromatographie (Laufmittel und Chloroform) getrennt und die Tryptanthrinzone gewonnen. Aus der erhaltenen Fraktion fiel nach Umkristallisieren aus Ethylacetat/Chloroform Tryptanthrin in Form von gelben Kristallen aus (35 mg).

### Beispiel 2

Tryptanthrin sowie die A- und D-ringsubstituierten Indolo[2,1-b]chinazolin-6,12-dione sind nach bekannten Methoden synthetisch zugänglich, zweckmässig nach der allgemeinen Synthesevorschrift von Mitscher L. et al, Heterocycles (1981), 14, 1017-1021. Aus entsprechend substituiertem Isatin und Isatoinanhydrid (Isatoic anhydride) entsteht unter Zugabe von NaH in trockenem TMF das entsprechende Tryptanthrinderivat. Die Synthese der substituierten Isatinderivate erfolgte nach den Vorschriften von Baker und Mitscher, Patent W095/13807. Die als Ausgangsstoffe notwendigen Anilinderivate waren kommerziell erhältlich oder nach bekannten Synthesen zugänglich (s. z.B. Furniss et al., Practical Organic Chemistry 5. Aufl., 1989; March, J., Advanced Organic Chemistry, 3. Aufl. 1985).

Die Isatoinanhydride wurden analog den Vorschriften von Baker und Mitscher hergestellt. Als Ausgangsstoffe dienten die entsprechenden 2-Aminobenzoesäurederivate. Die Umsetzungen sind in Fig. 1 durch die Reaktionsfolgen Equ. 1, Equ. 2 und Equ. 3 dargestelllt.

### Beispiel 3

Unter Anwendung des oben beschriebenen Syntheseweges wurden u.a. folgende Indolo[2,1-b]chinazolin-6,12-dione synthetisiert:

**Tabelle 1**

| Verbindung | | Substituenten R₁ bis R₈ | | | | | | |
|---|---|---|---|---|---|---|---|---|
| Nr. | 1 | 2 | 3 | 4 | 5 | 6 | 7 | 8 |
| 1 | H | H | H | H | H | H | H | H |
| 2 | H | Cl | H | H | H | F | H | H |
| 3 | H | Cl | H | H | H | NO₂ | H | H |
| 4 | H | CH₃O | CH₃O | H | H | H | H | H |
| 5 | H | Cl | H | H | H | H | H | H |
| 6 | H | NO₂ | H | H | H | H | H | H |
| 7 | H | H | H | H | H | Br | H | H |
| 8 | H | H | H | H | H | NO₂ | H | H |
| 9 | H | H | H | H | H | F | H | H |
| 10 | H | H | H | H | H | Cl | H | H |
| 11 | H | H | H | H | H | OCH₃ | H | H |
| 12 | H | H | H | H | H | CH₃ | H | H |
| 13 | H | H | H | H | H | l | H | H |
| 14 | H | CH₃ | H | H | H | H | H | H |
| 15 | CH₃ | H | H | H | H | H | H | H |
| 16 | H | H | H | CH₃ | H | H | H | H |
| 17 | H | F | H | H | H | F | H | H |
| 18 | H | Br | H | H | H | H | H | H |
| 19 | H | F | H | H | H | H | H | H |
| 20 | H | NH₂ | H | H | H | F | H | H |
| 21 | H | H | H | H | H | H | Cl | H |
| 22 | H | H | H | H | Cl | H | H | H |
| 23 | H | H | H | CH₃O | H | F | H | H |
| 24 | H | CH₃ | H | CH₃ | H | F | H | H |
| 25 | H | CH₃ | H | H | H | F | H | H |
| 26 | H | H | F | H | H | F | H | H |
| 27 | H | H | H | H | H | H | H | F |
| 28 | F | H | H | H | H | F | H | H |
| 29 | CH₃ | H | H | H | H | F | H | H |
| 30 | H | H | H | CH₃ | H | F | H | H |
| 31 | H | H | H | H | H | F | H | F |
| 32 | H | H | NMP | H | H | F | H | H |
| 33 | H | H | H | H | NMP | H | H | H |
| 34 | H | H | H | H | H | H | NMP | H |
| 35 | NMP | H | H | H | H | F | H | H |
| 36 | H | H | H | H | H | F | NMP | H |
| 37 | H | H | H | H | H | CO₂Et | H | H |
| 38 | H | H | H | F | H | F | H | H |
| 39 | H | H | H | H | H | F | F | H |
| 40 | H | H | F | H | H | F | NMP | H |
| 41 | H | H | H | H | H | F | 3-MP | H |
| 42 | H | H | H | H | H | F | 2-MNBP | H |
| 43 | H | H | H | H | H | H | NBP | H |
| 44 | H | H | H | H | H | F | NMP | F |
| 45 | H | H | H | H | H | H | P | H |
| 46 | H | F | F | H | H | F | H | H |
| 47 | H | F | NMP | H | H | F | H | H |
| 48 | Cl | H | Cl | H | H | l | H | H |
| 49 | Cl | H | H | Cl | H | l | H | H |
| 50 | H | l | H | H | H | CO₂Et | H | H |
| 51 | H | l | H | H | H | l | H | H |
| 52 | H | H | H | OCH₃ | H | l | H | H |
| 53 | H | H | H | OCH₃ | H | H | H | H |
| 54 | H | l | H | l | H | l | H | H |
| 55 | H | Br | H | Br | H | l | H | H |
| 56 | H | l | H | l | H | CO₂Et | H | H |
| 57 | H | H | H | H | H | Cl | H | CH₃ |
| 58 | H | F | NBP | H | H | l | H | H |
| 59 | F | Br | H | Br | H | l | H | H |
| 60 | Cl | Br | H | Br | H | l | H | H |
| 61 | F | Br | H | Br | H | H | H | H |
| 62 | H | F | 3-MNBP | H | H | l | H | H |
| 63 | H | H | H | H | H | CO₂Bn | H | H |
| 64 | H | F | P | H | H | 1 | H | H |
| 65 | H | H | H | OH | H | l | H | H |
| 66 | H | H | H | H | H | CO₂2'Octyl | H | H |
| 67 | H | F | 3-MP | H | H | l | H | H |
| 68 | H | H | H | OCH₃ | H | CO₂Et | H | H |
| 69 | H | H | H | OCH₃ | H | SO₂n-Octyl | H | H |
| 70 | H | H | H | OCH₃ | H | SO₂NMP | H | H |
| 71 | H | H | H | OCH₃ | H | CO₂2'-Octyl | H | H |
| 72 | H | H | H | OCH3 | H | CO₂H | H | H |
| 73 | Cl | H | Cl | H | H | FmocAG | H | H |
| 74 | Cl | H | Cl | H | H | H | H | H |
| 75 | Cl | H | Cl | H | H | AAGOCH3 | H | H |
| 76 | H | H | H | OCH₃ | H | 1-Octenyl | H | H |
| 77 | H | F | 3-MP | H | H | CO₂H | H | H |
| 78 | H | 1-Octenyl | H | H | H | Cl | H | H |
| 79 | H | l | H | H | H | Cl | H | H |
| 80 | H | Octyl | H | H | H | Cl | H | H |
| 81 | H | OPO₃Na₂ | H | H | H | H | H | H |
| 82 | H | OH | H | H | H | H | H | H |
| 83 | H | H | F | H | H | Cl | H | H |
| 84 | H | H | H | Obn | H | F | H | H |
| 85 | H | H | NME | H | H | Cl | H | H |
| 86 | H | H | 4-M | H | H | Cl | H | H |
| 87 | H | H | Piperdin | H | H | Cl | H | H |
| 88 | H | Octyl | H | H | H | H | H | H |
| 89 | H | 6-Acoctenyl | H | H | H | Cl | H | H |
| 90 | H | NEDME | H | H | H | Cl | H | H |
| 91 | H | 6-Acoctyl | H | H | H | Cl | H | H |
| 92 | H | H | H | OH | H | F | H | H |
| 93 | H | H | DPA | H | H | Cl | H | H |
| 94 | H | H | H | OCH₃ | H | cis-1-Octenyl | H | H |
| 95 | H | 2-AG | H | H | H | Cl | H | H |
| 96 | H | H | H | OCH₃ | H | Octyl | H | H |
| 97 | H | H | H | H | H | CF₃ | H | H |
| 98 | H | H | NMEP | H | H | Cl | H | H |
| 99 | H | H | SAR | H | H | Cl | H | H |
| 100 | H | H | NME Sacchester | H | H | Cl | H | H |
| 101 | H | H | H | H | H | OCF₃ | H | H |
| 102 | H | H | NME Octylester | H | H | Cl | H | H |
| 103 | H | H | H | H | H | n-Octyl | H | H |
| 104 | H | H | F | H | H | n-Octyl | H | H |
| 105 | H | H | n-Octyl | H | H | n-Octyl | H | H |
| 106 | H | H | NME | H | H | F | H | H |
| 107 | H | H | NME | H | H | n-Octyl | H | H |
| 108 | H | H | NMP | H | H | n-Octyl | H | H |
| 109 | H | H | F | H | H | OCF₃ | H | H |
| 110 | H | H | NME | H | H | OCF₃ | H | H |

In der obigen Tabelle 1 haben die Abkürzungen folgende Bedeutung

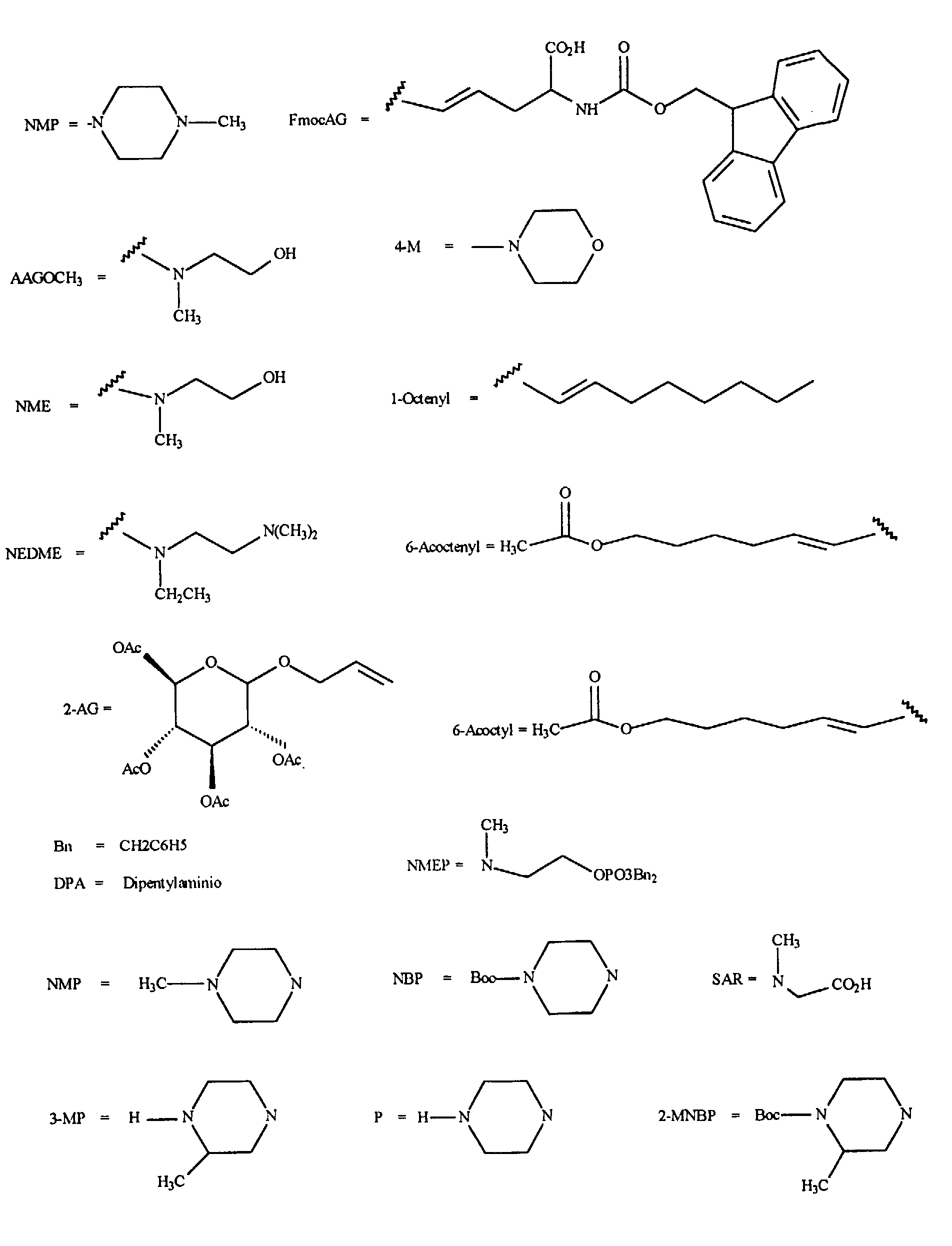

### Beispiel 4

### Biologische Aktivität von Tryptanthrin in vitro

Die Bestimmung der COX-2 inhibitorischen Wirkung der gemäss Beispiel 2 synthetisierten Verbindungen wurde mit Mono Max 6-Zellen nach der Methode von Berg et al., J. Pharmaco. Toxicol. Methods 37, 179-186 (1997) durchgeführt.

Dabei wurden Mono Mac 6-Zellen mit unterschiedlichen Konzentrationen der Testsubstanzen während 30 min inkubiert, anschliessend Lipopolysaccharid (LPS, 100 ng/ml) zupipettiert und während weiteren 6 Stunden inkubiert. Anschliessend wurde Arachidonsäure zugegeben (Endkonz. 30 µM), während 15 Min. inkubiert und das gebildete Keto PGF 1α mittels ELISA bestimmt.

In einer Variante wurden Testsubstanz und LPS zeitgleich eingesetzt. Die Messung der COX1 Hemmung erfolgte in HEL-Zellen nach der Methode von Berg et al., J. Pharmacol. Toxicol. Methods 37, 179-186 (1997). Dabei wurden HEL Zellen mit unterschiedlichen Konzentrationen der Testsubstanzen während 30 Min. inkubiert, anschliessend Arachidonsäure zugesetzt (Endkonz. 30 uM), während 15 Min. inkubiert und das gebildete Tromboxan B2 mittels ELISA gemessen.

Die in Beispiel 2 hergestellten Substanzen wurden in zellfreien und zellulären Testsystemen nach bekannten Methoden auf NF-kB hemmende Wirkung geprüft. Dabei wurde im wesentlichen nach der von Rüngeler, P. et al, Planta Medica 64, 588-593 (1998), beschriebenen Methode verfahren. Bestimmt wurde die in-vivo und die in-vitro NF-kB Bindung an DNA durch Messung der elektrophoretischen Mobilitätsverschiebung.

Die in vivo Bindung wurde mit Jurkat T-Zellen durchgeführt. Die Zellen wurden während einer Stunde mit verschiedenen Konzentrationen der verschiedenen Extrakte inkubiert und anschliessend mit TNF-a während einer Stunde stimuliert. Total-Protein-Extrakte der Zellen wurden gewonnen und in einem Assay auf elektrophoretische Mobilitätsverschiebung (electrophoretic mobility shift asasy, EMSA) auf NF-kB-bindende Aktivität geprüft.

Die in vitro Bindung wurde ebenfalls mit Jurkat T-Zellen geprüft. Zellextrakte von stimulierten wie unstimulierten Zellen wurden gewonnen. Gleiche Proteinmengen (10-20 µg) wurden 2 Stunden mit Konzentrationsreihen der verschiedenen Isatis-Extrakte inkubiert und schliesslich auf elektrophoretische Mobilitätsverschiebung (EMSA) analysiert. Daten ausgewählter Verbindungen sind in Tabelle 2 aufgelistet.

**Tabelle 2**

| Verbindung | COX-1 | COX-2 | NF-kB |
|---|---|---|---|
| 1 | 2 | 0,04 | 1 |
| 5 | 10 | 2 | 50 |
| 10 | 1 | 0,5 | 5 |
| 20 | 5 | 0,5 | 5 |
| 24 | >50 | >50 | >100 |
| 32 | 1 | 1 | 10 |
| 37 | >50 | >50 | >100 |
| 42 | >50 | >50 | >100 |
| 56 | * | * | * |
| 60 | * | * | * |
| 69 | >50 | >50 | >100 |
| 86 | 5 | 0,01 | 0,5 |
| 99 | 5 | 0,5 | 1 |
| 100 | 10 | 0,02 | 5 |
| 110 | 2 | 0,5 | 5 |
| COX-1 und COX-2: IC 50 (µM/Liter) NF-kB: Grenzkonzentration (µM/Liter), bei der eben noch ein Hemmeffekt im EMSA feststellbar ist )* Cytotoxisch bei den geprüften Konzentrationen | | | |

Zusammenfassend bietet die Erfindung ein Mittel zur Hemmung des Transkriptionsfaktors NF-kB mit gleichzeitiger direkter Hemmwirkung auf Cyclooxygenasen, wobei ausgewählte Verbindungen wie 1 und 86 eine deutliche präferentielle Hemmung der induzierbaren COX-2 aufweisen. Die Indolo[2,1-b]chinazolin-6,12-dione sind demnach eine neue Klasse von anti-inflammatorischen Wirkstoffen mit einem speziellen dualen Wirkmechanismus, nämlich auf der Ebene der Transkription und auf derjenigen der Eicosanoid-Synthese.

Auswählte Indolo[2,1-b]chinazolin-6,12-dione, wie z.B. bevorzugte Verbindungen der Formel (1), bei der alle R₁ bis R₈ Wasserstoffatome sind, und bevorzugte Verbindungen der Formel (1), in denen R₁,R₂ = R₄,R₅ = R₇,R₈ = H, R₃ ein Stickstoff enthaltender Substituent, wie 4-M, und R₆ Halogen, insbesondere Cl ist, bieten aussichtsreiche Mittel zur Behandlung von Zuständen, deren Zusammenhang mit der Aktivierung von NF-kB und/oder der Induktion der Bildung von COX-2 bereits bekannt ist, vermutet wird oder noch festzustellen ist, insbesondere als antiphlogistische bzw. antirheumatische Mittel, zur präventiven oder adjuvanten Behandlung von morbus Alzheimer und von Krebserkrankungen sowie zur präventiven oder beziehungsweise adjunktiven Dauermedikation bei Altersbeschwerden. Zu den bevorzugten Verbindungen der Formel (1) gehören ferner die Verbindungen, die bei der in Beispiel 4 beschriebenen Prüfung NF-kB-Werte ≤ 10, insbesondere ≤ 5, COX-2 Werte ≤ 1, insbesondere ≤ 0,05, und COX-1 Werte ≤ 10, vorzugsweise ≤ 5 aufweisen.

Allgemein liegt die Auswahl der für eine gegebene Behandlung optimalen Verbindung der Formel (1) bzw. des entsprechenden pharmazeutisch akzeptablen Salzes aufgrund der vorstehenden Erläuterungen im Rahmen des Wissens der Fachleute.

## Patentansprüche

1. Indolo[2,1-b]chinazolin-6,12-dione der Formel (1) 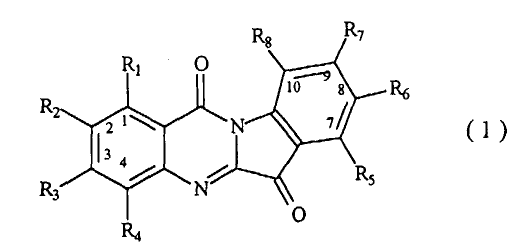 in der R₁ bis R₈ unabhängig voneinander gewählt sind aus Wasserstoffatomen und pharmazeutisch akzeptablen Substituenten, und pharmazeutisch akzeptable Salze von Verbindungen der Formel (1) zur Verwendung für die Herstellung von Arzneimitteln zur Hemmung des Transkriptionsfaktors NF-kB und/oder zur Hemmung von COX mit einer präferentiellen Hemmwirkung auf COX-2.

2. Indolo[2,1-b]chinazolin-6,12-dione der Formel (1) 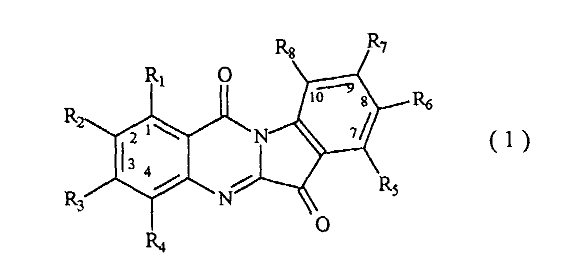 in der R₁ bis R₈ unabhängig voneinander gewählt sind aus Wasserstoffatomen und pharmazeutisch akzeptablen Substituenten, und pharmazeutisch akzeptable Salze von Verbindungen der Formel (1) zur Verwendung für die Herstellung von Arzneimitteln mit antiphlogistischer, insbesondere antirheumatischer Wirkung zur Behandlung von entzündlichen Erkrankungen, insbesondere des rheumatischen Formenkreises und der Arthritiden.

3. Indolo[2,1-b]chinazolin-6,12-dione der Formel (1) 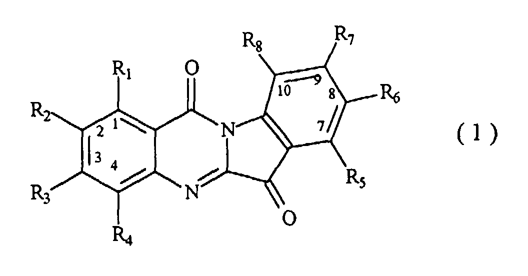 in der R1 bis R8 unabhängig voneinander gewählt sind aus Wasserstoffatomen und pharmazeutisch akzeptablen Substituenten, und pharmazeutisch akzeptable Salze von Verbindungen der Formel (1) zur präventiven oder adjuvanten Behandlung von morbus Alzheimer.

4. Indolo[2,1-b]chinazolin-6,12-dione der Formel (1) 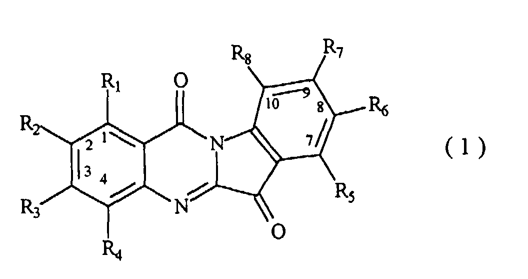 in der R₁ bis R₈ unabhängig voneinander gewählt sind aus Wasserstoffatomen und pharmazeutisch akzeptablen Substituenten, und pharmazeutisch akzeptable Salze von Verbindungen der Formel (1) zur Verwendung für die Herstellung von Arzneimitteln zur präventiven oder adjuvanten Krebsbehandlung.

5. Indolo[2,1-b]chinazolin-6,12-dione der Formel (1) 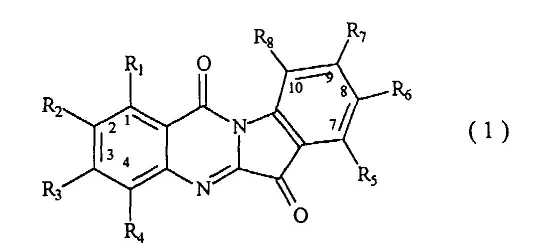 in der R₁ bis R₈ unabhängig voneinander gewählt sind aus Wasserstoffatomen und pharmazeutisch akzeptablen Substituenten, und pharmazeutisch akzeptable Salze von Verbindungen der Formel (1) zur Verwendung für die Herstellung von Arzneimitteln für die präventive oder adjuvante Dauermedikation bei Altersbeschwerden.

6. Arzneimittel, dadurch gekennzeichnet, dass es mindestens ein Indolo[2,1-b]chinazolin-6,12-dion der Formel 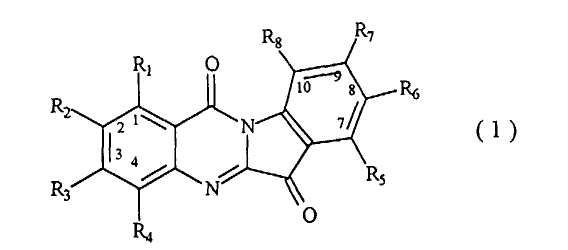 in der R₁ bis R₈ unabhängig voneinander gewählt sind aus Wasserstoffatomen und pharmazeutisch akzeptablen Substituenten, und/oder mindestens ein pharmazeutisch akzeptables Salz einer Verbindung der Formel (1) enthält, zur Hemmung des Transkriptionsfaktors NF-kB und/oder zur Hemmung von COX mit präferentieller Hemmwirkung auf COX-2.
